# EUROPEAN PATENT SPECIFICATION

(11) **EP 1 275 357 B1**
(45) Date of publication and mention of the grant of the patent: **02.03.2011**
(21) Application number: 02022497.8
(22) Date of filing: 10.04.1997
(51) Int. Cl.: A61F 5/445

(54) **Customized ostomy devices**
Angepasste Ostomievorrichtungen
Appareils d'ostomie adaptés

(30) Priority: 10.04.1996 US 631767
(43) Date of publication of application: 15.01.2003
(62) Divisional of application: 97201073.0
(73) Proprietor: ConvaTec Technologies Inc., Las Vegas, NV 89169 (US)
(72) Inventor: Cline, John B, New Brunswick, New Jersey (US); Konicky, Philip J, Newtown, Pennsylvania (US); Johnsen, Kenneth A, Piscataway, New Jersey (US); Kuczynski, Thomas J, Sayreville, New Jersey 08872 (US)
(74) Representative: Mays, Julie

(56) References cited:
- EP-A- 0 574 098
- WO-A-92/18074
- DE-A- 4 015 186
- GB-A- 2 188 085
- US-A- 5 429 626

## Description

The present invention relates to ostomy devices of the type including a waste collection pouch adapted to be adhesively affixed to the skin surrounding the stoma and more particularly to a method for fabricating ostomy devices customized to the stomal topography and preferences of the user.

Certain surgical procedures known as colostomy, ileostomy and urostomy result in an opening in the abdominal wall, called a stoma, which permits waste discharge from the interior of a body cavity. Since the patient has no control over the waste discharge, it is often necessary for the patients who have undergone these surgical procedures to utilize an ostomy device to protect the stoma and collect the waste material as it is discharged.

Over the years, ostomy devices of a variety of different types and constructions have been utilized. Various materials and adhesives have been developed to increase the utility and wearability of the devices.

The basic device includes a collection receptacle or pouch connected to an adhesive coated wafer (also called a faceplate or label) which serves to mount the pouch to the body proximate the stoma. The pouch includes first and second thin barrier film walls which are sealed by heat welding or the like along the periphery. The pouch has an inlet opening defined by a plastic attachment ring which is designed to align with the stoma and an outlet for emptying material from the pouch. In single piece devices that do not form part of the invention, the attachment ring of the pouch is permanently affixed to a corresponding plastic attachment ring affixed to the adhesive coated wafer. In two piece devices according to the invention, the pouch is detachably mounted to the wafer by employing detachable rings, permitting the pouch to be replaced without removal of the wafer.

Wafers and pouches are manufactured in a variety of different standard shapes, sizes and configurations to meet the many different needs of the users. While these standard products meet the needs of the average user, they do not ideally meet the needs of any particular individual.

In most cases, the user must modify the product prior to use to suit his or her anatomy or lifestyle. Typical modifications which can be performed include cutting of the stoma receiving opening in the wafer, trimming the outside of the wafer, addition of convex inserts, application of paste and filters and folding or trimming the pouch.

DE-A-4 015 186 discloses modification of a preformed, one-piece product.

US-A-5429626 discloses a two-piece device customised for a particular size stoma.

However, even these modifications may not result in an ideal product for the particular individual. Users may have difficulty in performing the modifications, as well. This may be due to poor dexterity, poor eyesight or diminished mental capacity. Some desired modifications may be very difficult or impossible without special equipment. Some users may see making such modifications as taking too much time or as an undesirable task.

Thus, because of difficulties or simple reluctance on the part of the user, the modifications to the products are done poorly. As a result, product performance may suffer.

It is the general object of this invention to eliminate the above problems by fabricating ostomy devices which are customized so as to be uniquely suited to the needs and preferences of each individual user. This object may be achieved in a variety of different ways, using technologies of different sophistication and cost.

As the first step, the topography of the stomal area of the patient is carefully measured. This can be done manually, as the user is examined by an Enterostomal Therapist, photographically or by casting. It can be done by computerized touch probe, magnetic image resonance imaging (MRI), computerized axial tomography (CAT scan) or by laser scanning.

The resulting information is then recorded. Measurements taken by a therapist or other professional can be stored by marking a card or forming a template by a tracing or cutout of the stoma opening and wafer outline. Information in this form is later converted into a digital image. Photos taken at different angles can be digitized to form an electronic image of the anatomy. Computerized touch probe, MRI and CAT scan equipment provide similar electronic size and shape maps. A laser scanner can be employed to provide 3-dimensional shape information either of the body itself or of a cast made of the body.

The digital information is placed in a database file. When an order is received, the information is retrieved from the file and used to determine of physical attributes of the customized wafer and pouch. For example, the size, shape and location of the stoma receiving opening in the wafer, the wafer outline, adhesive type and surface contour can be selected. The pouch size, shape, tail location, tap type, tap location, flange style and filter location can all be choosen.

Once the physical attributes are determined, the appropriate tooling is selected and adjusted by the computerized controller for the fabrication equipment. Cutting of the wafer hole and outline is done with the cutting tool mounted onto an X-Y table. The cutting tool itself could be a laser, a high pressure water jet, a hot wire, a high speed grinding/machine tool, a knife, a saw, a RF scalpel or be accomplished by nibbling or punching. The desired surface contour of the wafer may be achieved by vacuum forming barrier adhesive over filler material molded into the required shape, over a base formed by multiple slices joined together to approximate the surface contour or over a planar base upon which protrusions of various shapes are affixed and into which valleys are formed. Convex disks and inserts can be used and if necessary supplemented with shape altering material.

Pouches are customized by selecting the outline configuration and welding the periphery by a laser, ultrasonic horn, hot air weld nozzle or hot roller mounted on an X-Y table. Filters and taps can be welded at desired locations.

After the order is completed, the products may be labeled with the name or code number of the customer. The products are then packed and shipped to the customer in accordance with instructions from the customer. The topographical, preference information and patient history are kept in the electronic file for future applications. Stoma site examination is only required once, except that stoma site changes may require additional examination.

In accordance with the present invention the ostomy device of claim 1 is provided. The ostomy device customized for a particular user is provided including an adhesive wafer with a stoma receiving opening. An attachment ring is mounted on the wafer and defines the area in which opening is situated. A waste collection pouch is provided having an inlet. Means are provided for mounting the pouch on the wafer with the inlet aligned with the opening. The size, shape and location of the stoma receiving opening within the defined area is selected in accordance with the size, shape and location of the stoma of the user.

The wafer has a shape and size selected in accordance with the topography of the stomal area of the user.

The pouch has a contour selected in accordance with the topography of the stomal area of the user. The pouch includes an attachment ring. The location of the attachment ring on the pouch is selected in accordance with the topography of the stomal area of the user.

The surface contour of the wafer is selected in accordance with the topography of the stomal area of the user.

In accordance with these objects and those which may hereinafter appear, the present invention relates to customized ostomy devices as set forth in detail in the following specification and recited in the annexed claims, taken together with the accompanying drawings, wherein like numbers relates to like parts and in which:
Fig. 1 is a schematic drawing of an overall method usable for the present invention;
Fig. 2 is a schematic drawing illustrating typical prior art one piece and a two piece customized ostomy product according to the invention which can be obtained using the method;
Fig. 3 is a schematic diagram illustrating the features of the ostomy device which can be customized;
Fig. 4 is a plan view of a typical card upon which measurement information can be recorded;
Fig. 5 illustrates three parts used to simulate a customized wafer, prior to cutting;
Fig. 6 is an exploded view of the parts illustrated in Fig. 5, after cutting;
Fig. 7 is an isometric view of the assembled parts of Fig. 6;
Fig. 8 illustrates the steps in making a mold of the peristomal area;
Fig. 9 is an isometric view of a wafer with a customized stoma receiving opening;
Fig. 10 is a plan view of a wafer with a customized contour;
Fig. 11 is an isometric view of a wafer with a customized surface contour;
Fig. 12 is a plan view of a wafer mold built of multiple slices;
Fig. 13 illustrates the slices which make up the mold of Fig. 12;
Fig. 14 is a cross-sectional view of a wafer with a conventional convex disk;
Fig. 15 is a wafer with a surface contour formed by injecting shaping material;
Fig. 16 is a cross-sectional view of a wafer with an annular pouch filled with corn starch;
Fig. 17 is a cross-sectional view of a wafer with an annular pouch as it is filled with a shaping liquid;
Fig. 18 is a partial cross-sectional view of a wafer with a compressed sponge material in the pouch;
Fig. 19 is a view similar to Fig. 18 with the sponge expanded;
Fig. 20 is a partial cross-sectional view of the wafer with the injection needle in place;
Fig. 21 is a partial cross-sectional view of the wafer with the valve closed;
Fig. 22 is a partial cross-sectional view of a wafer with an integral chamber before it is closed;
Fig. 23 is a partial cross-sectional view of the wafer of Fig. 22 with the chamber closed and filled;
Fig. 24 illustrates pouch sealing by laser;
Fig. 25 illustrates pouch sealing by hotwheel;
Fig. 26 is a side elevational view of a part welder;
Fig. 27 is a plan view of the film showing three different pouch configurations;
Fig. 28 is an elevational view of a portion of a pouch with minimal head room;
Fig. 29 is an exploded view of a one piece device which does not form part of the invention;
Fig. 30 is an exploded view of a pouch with a flange having a channel adapted to receive a detachable mating ring;
Fig. 31 is a plan view of a pouch with an attachment ring and flange;
Fig. 32 is an exploded view of a ring with a channel and a snap-fit type flange;
Fig. 33 illustrates one version of the snap-fit structure;
Fig. 34 illustrates a second version of the snap-fit structure;
Fig. 35-40 show different structures of a two part attachment ring usable for the invention;
Fig. 41 shows three different tap types and a washer for mounting same;
Fig. 42 is a cross-sectional view of a nonrotary tap mounted on the washer;
Fig. 43 is a cross-sectional view of a rotary tap type mounted on the washer;
Fig. 44 illustrates a first type of a tail clip;
Fig. 45 illustrates the custom cutting of the clip of Fig. 44;
Fig. 46 illustrates a second type of a tail clip; and
Fig. 47 illustrates the custom cutting of the clip of Fig. 46.

In general, the present invention presents a radically different approach to ostomy device design in which the object is to produce appliances which are customized to the anatomy and preferences of the user. Thus, instead of producing wafers and pouches in a variety of standard sizes and shapes and attempting to make minor modifications in the field, aimed at meeting the needs of theoretical "average" patient, the ostomy devices of the present invention will be custom designed and fabricated to suit the needs of the individual. No modification will be necessary in the field and optimum results and comfort are achievable.

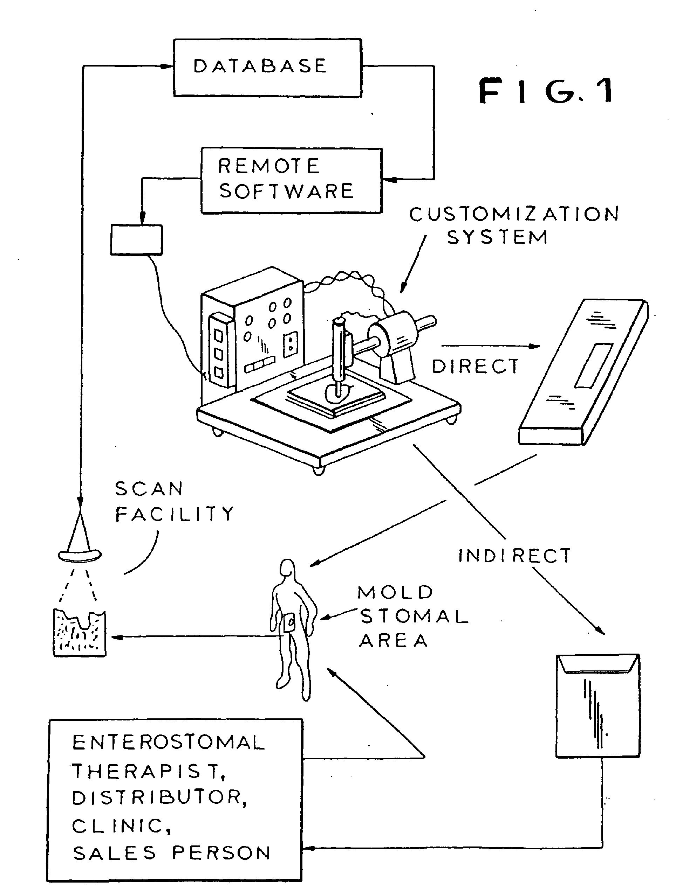

An overall method is illustrated schematically in Figure 1. It begins with an examination of the patient to obtain measurements of the stomal area. The measurement information is then recorded. In the figure, this is represented by taking a mold of the stomal area. This can be done by the Enterostomal Therapist, a salesperson with the appropriate skill at a clinic or the like. However, as described below, may other methods of measurement and recordation are possible.

The mold is taken to a facility where it is scanned, such by a laser scanner. The scanner forms a digital image or map of the contours of the mold material, which of course represents the anatomy of the patient. The digital image is stored in an electronic patient file in a database.

The digital image and other patient information, such as patient history and preferences are kept in the file. The appropriate software and the file information are loaded into a computerized controller, which determines the physical attributes of the wafer and pouch in accordance with the recorded information. The controller selects and adjusts the appropriate tools tc perform the fabrication work and guides the machines, such as the computerized laser cutter illustrated, to perform the fabrication operations. The laser cutter is illustrated cutting the stoma receiving opening in a wafer.

Once fabrication of the wafers and pouches are complete, they are packaged and shipped either directly to the customer, or to the therapist, salesperson or clinic responsible for examining the patient and distribution to the patient.

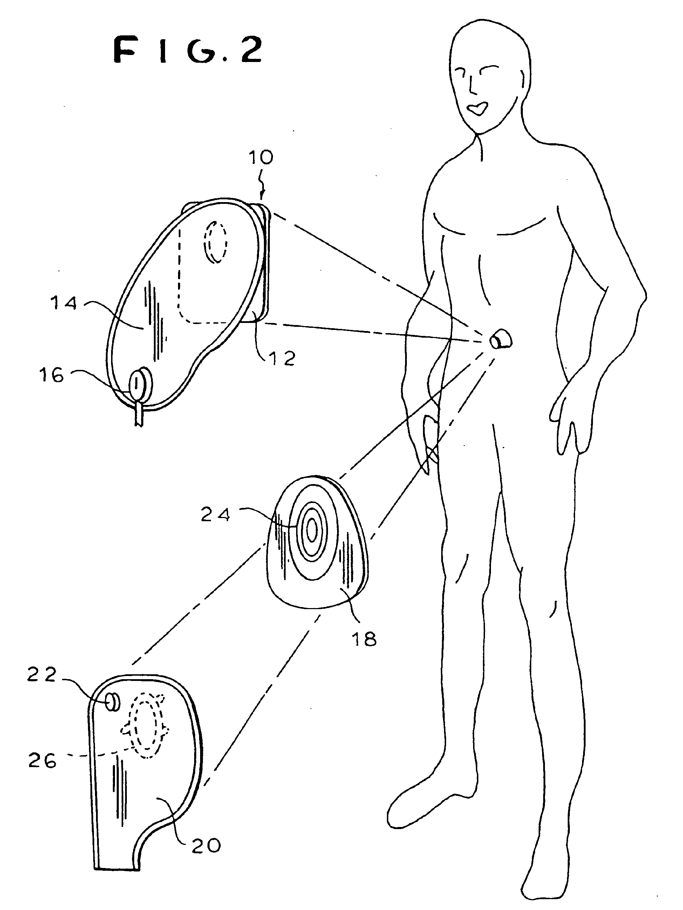

Figure 2 illustrates a patient with a stoma. Two different versions of the customized ostomy device, a one piece device and a two piece device, are shown. The one piece ostomy device 10 consists of a generally rectangular adhesive wafer 12 and a pouch 14 connected thereto. Pouch 14 has a customized contour and a rotatable drain tap 16. The two piece applicance according to the invention is also shown. The two piece appliance comprises a wafer 18 with a customized contour and a pouch 20 with a filter 22. Wafer 18 has a coupling ring 24 which is adapted to detachably mount to a mating coupling ring 26 affixed to pouch 20.

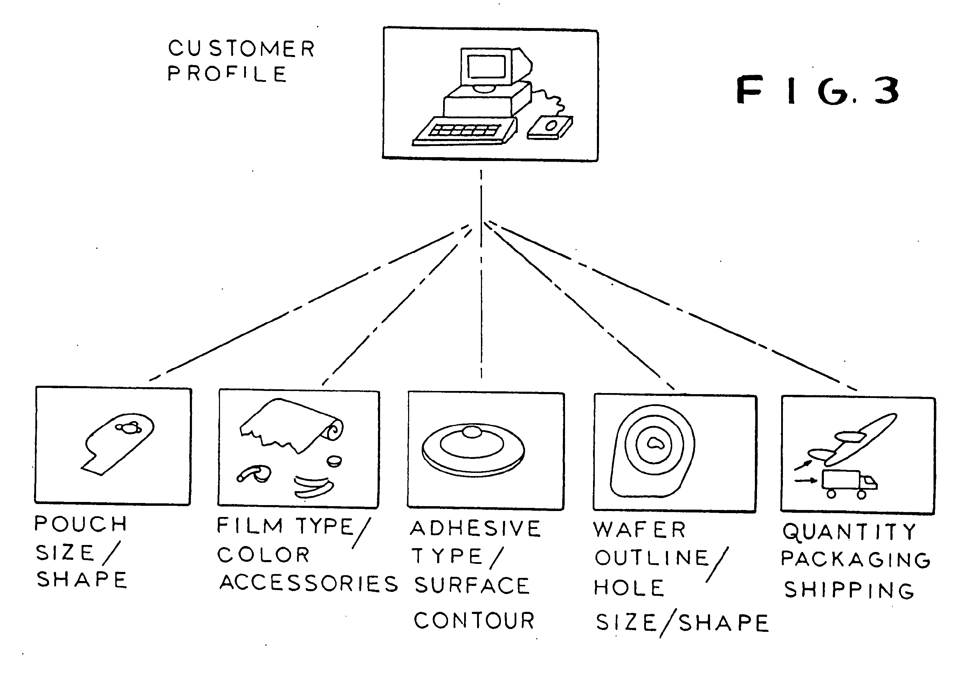

Figure 3 schematically illustrates the types of design features which could be customized. The customer profile is stored in a computer database. The profile may include a digital image of the patient's anatomy, the patient's history, including any allegeries to particular adhesives or films and preferences for adhesives, color, type of appliance (one piece or two piece) , quantity for each order, packaging and shipping methods.

With this information on file, the computer can determine the physical attributes of the customized wafer and pouch. The pouch size and shape can be selected, along with the film type, color and required accessories. The type and contour of the wafer adhesive can be determined. The shape, size and location of the stoma receiving hole in the wafer and the wafer outline can be chosen.

Moreover, the quantity required can be calculated based on past order frequency. How the devices are to be packed and the method of shipping are also determined.

Once these decisions are made, the information is used with the appropriate software to select the appropriate materials, fabrication tools and to guide the fabrication tools. It is used to determine the quantities to be made, how many are to packed and how they are to be shipped.

### MEASUREMENT OF THE STOMAL AREA

Regardless of the fabrication method, the first step is an examination of the patient and careful measurement of the contour of the stomal area to develop a digitized 2-dimensional or 3-dimensional image or map of the topography of the anatomy of the patient. Measurement, recordation and digitization of the measurements can be done in a variety of different ways.

One simple method is to measure the stomal area manually and record the measurements on a card. The card allows the ostomate or professional to convey measurement information to an automated customized manufacturing system in an efficient and convenient manner. The recorded information may include the attributes of the size and shape of the stoma receiving hole in the adhesive wafer, along with the wafer outline size and shape, the relation between them and the relation of same with other components. The card may also display the limits of the system for particular products and sizes, and the optimum size or product which is best suited for the application.

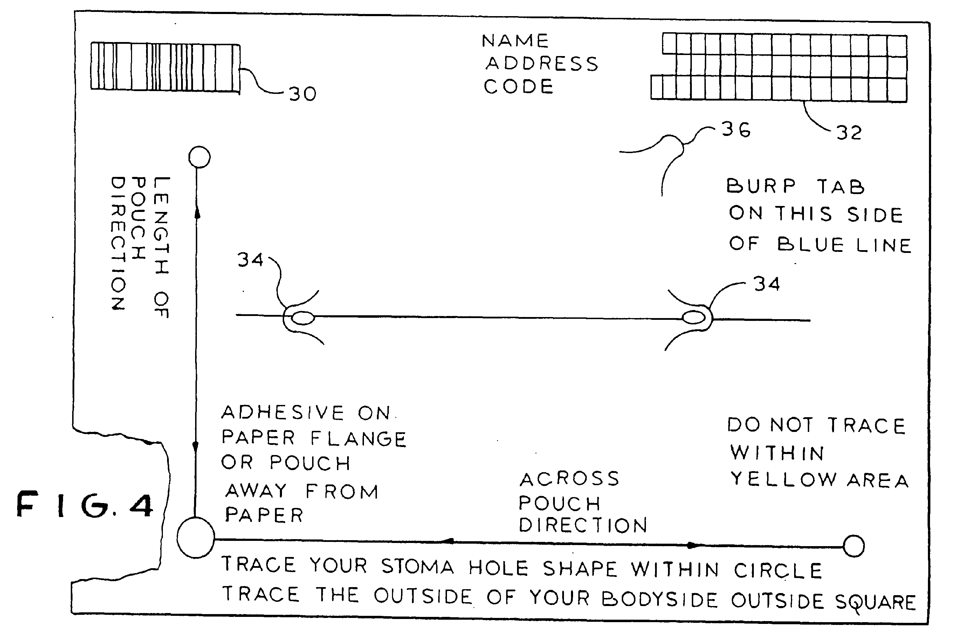

Some of the features of a typical card are illustrated in Figure 4. The card generally designated 28 may be composed of paper, foil, plastic, film or composites of same which can be subjected to mailing, shipping or transmitting without damage or loss of accuracy. Detachable copies of the card may be made to permit the user or professional to retain a record of the information.

The information on the card may be formed by drawing or tracing the stoma size, shape and location and the relationships between the stoma and other physical attributes of the anatomy. This may be done with a special pencil, pen or marker which enables the card to be automatically read. Another approach is to cut a hole can be cut into the card to indicate the size, shape and location of the stoma.

An adhesive wafer or a similarity thereof could be cut as desired and applied to the card. A special tape, foil or film could be cut and applied to the card. Even a piece of paper simulating a wafer could be cut and applied to the card. Components of the card could be assembled in a manner representing the desired size, shape and location of the components of the product.

Card 28 may carry a distinct bar code type label 30 which identifies the patient. The label is read by the system to automatically index the attributes in a computer file. The label is shown as being located in the upper left of the card.

An area 32 for the patient's name, address and other information may be provided. This area is read by the system, the information is stored and may be used to generate a shipping label. Area 32 is shown as being situated in the upper right of the card.

Location holes or spots which are used by the system as datums for measuring size, shape and relationships of attributes may be provided on the card. The scheme of the holes orient and scale the information on the card for automatic processing by the system. These can be located near the bar code label in the lower left and lower right of the card.

Various symbols may be used on the card to guide the user to furnish attribute information accurately. These may include representations of product characteristics such as belt attachments on flange tabs 34; burp tabs 36, colored, shaded or outlined areas; colored lines or instructional notes. These may be located near the center or lower edge of the card.

Special inks which are detected or not detected by the system as part of the automated reading processes may be used to print the symbols. Any of the features may also be punched, cut, laminated, coated or embedded.

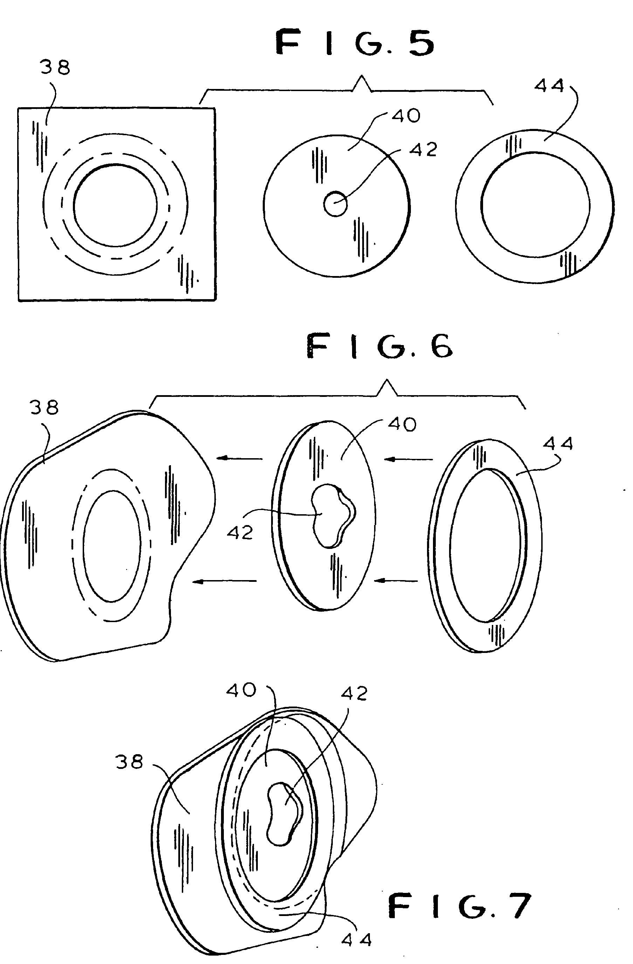

Another approach, which is illustrated in Figs. 5, 6 and 7 would be to provide a template of simulated components stamped from a sheet of a self-adhering plastic, such as vinyl. The user would remove each components, one at a time, from the sheet and cut or trim the simulated component to indicate the desired characteristics of the wafer.

The main component would be a clear square sheet 38 which could be trimmed to the exact size and shape desired by the end user as shown in Fig. 6. Another component would be a disk 40, made of the same material, which would feature a small central "starter hole" 42. This hole 42 could be trimmed to the exact size and shape required by the user to fit his/her stoma. Once trimmed, this component could be attached to the first component 38 in the location and orientation desired by the user. In a similar manner, a component 44 representing the attachment ring could be mounted on the first two components to represent the desired flange placement. In a similar manner, other features such as border material could also be simulated, trimmed to the desired shape and size, and located on the assembly as required by the user. Contrasting colors of each component would enable the size, shape, and relative location of all of the components to be scanned and recorded. Furthermore, the nature of the assembly and the materials used would allow the user to "trial fit" the assembly to ensure proper fit.

The information on the card or template of simulated components must be read and converted into digital form for storage and use by the system. This could be done manually, by an operator observing the card or simulated components and entering data into a computer via a keyboard. A mechanized reading method could be used as could electronic, magnetic or light sensing techniques, or some combination thereof.

Somewhat more sophisticated measuring techniques are also available. One perferred method is to make a mold of the peristomal area and thereafter scan the mold to create a digital image.

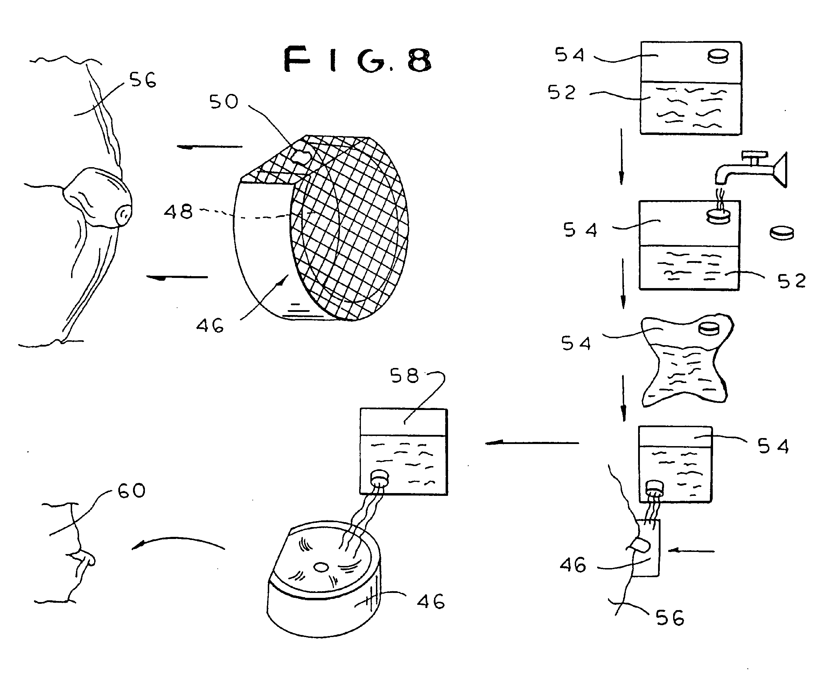

As illustrated in Fig. 8, a soft foam "horse collar" 46, composed of plastic mesh can be constructed with an open side 48 and an inlet 50 in the top surface. A pre-mixed aliginate 52 in a plastic bag 54 is provided. Water is introduced into the bag 54 and the material is kneaded to the required consistancy.

Holding form 46 against the body 56 with the stoma received in the open side 48, the mold material is poured in the opening 50 in the top of the form 46 and permited to harden. The mold 46 is then removed and pre-mixed plaster is poured in from a bag 58 and allowed to harden to form a plastic reproduction 60 of the stomal area. The plaster reproduction could be transported to another location where a laser scanner is employed to obtain a digital image. The plaster reproduction could even be used directly to create the product, if required.

Another measurement possibility is to use photographic images. Photographs of the stoma could be taken at different angles. Three dimensional information as to the size and shape of the stoma could thus be recorded. The photographies could be transferred to a facility to be digitized to form an electronic image.

Computerized touch probing with a pencil shaped touch probe interfaced with a personal computer could be employed. The probe touches many locations around the stoma. At each point, the location relative to the other touch points is recorded in the computer. The electronic size and shape map of the stoma site formed in this way can be transferred and used in the system.

Magnetic resonance imaging (MRI) and computerized axial tomography (CAT Scan) can be also used. MRI and CAT equipment is widely available. Both can provide an electronic size and shape map of the stoma cite suitable for use in the system.

Laser scanning can also be employed. Laser scanning of the stoma site (or a plaster reproduction) can provide an electric file with 3-dimensional size and shape information for use in the system.

### STORAGE OF THE MEASUREMENT INFORMATION

Depending upon how the measurements are taken, the information is read, if in card form, or scanned, if in the form of a simulation, a plaster cast or photographs and digitized. MRI, CAT scan, touch probe and laser scanning equipment may generate a digitized image directly.

The digitized measurement data is electronically transferred to a customer file database. In addition, other pertinent information such as customer preferences and history of product usage or allergies to certain materials is recorded into the database as well.

### UTILIZING THE DIGITIZED INFORMATION TOD ETERMINE PHYSICAL ATTRIBUTES OF THE DEVICE

Once all of the information has been recorded and digitized, it is stored in a customer file database. Each time an order from a particular user is received, the user's file is retrieved and the data downloaded into a computer which has the appropriate software to select the physical attributes of the customized wafer and pouch to be fabricated.

Such features which can be customized include the size, shape and location of the stoma receiving opening in the wafer, the wafer outline, the adhesive type and surface contour. With regard to the pouch, the size, shape, tail location, tap type and location and filter location can be choosen. In addition, accessories such as clamps or belts can be designated for inclusion in the packaging.

### FABRICATION OF THE OSTOMY DEVICE

Once the physical attributes of the ostomy device have been selected, this information is downloaded into a computerized equipment controller which selects and adjusts the required tooling. Thus, a tool for cutting the stoma receiving hole in the wafer and wafer outline is selected and mounted on an X-Y table to be guided through the cutting process. Likewise, a tool for welding the pouch periphery is mounted on an X-Y table. Equipment for affixing the attachment ring, tap and filter to the pouch wall is selected as well.

The controller directs each operation and fabricates the desired number of wafers and pouches of the selected materials. It then causes the wafers, pouches and accessories to be packed and shipped.

### CUSTOMIZATION OF THE ADHESIVE WAFER

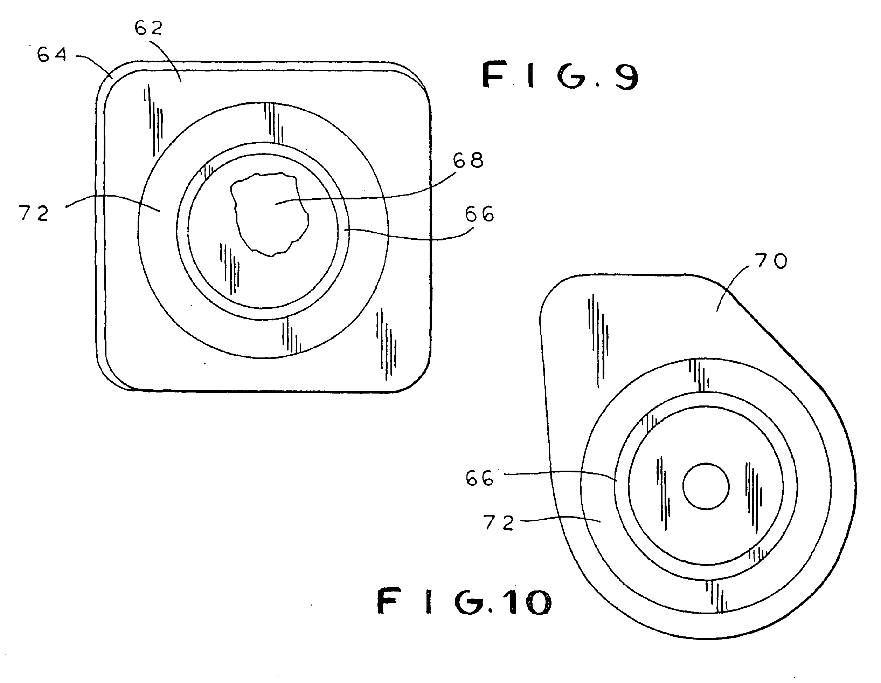

The wafer illustrated in Fig. 9 consists of a backing 62 which may be made of a non-woven polyethlyene material. A pressure sensitive adhesive layer 64 is cast onto the backing. The adhesive layer 64 is preferably 0.1 mm (4 mils) thick and can be an acrylic microporous adhesive as taught by Copeland in U.S. Patent No. 3,121,021, a microporous hydrocolloid adhesive as taught by Cilento in U.S. Patent No. 4,427,727, or a polyisobutylene - hydrocolloid containing adhesive as taught by Chen in U.S. Patent No. 3,339,546, by Chen et al. in U.S. Patent No. 4,192,785, by Pawelchak in U.S. Patent No. 4,393,080, or it can be adhesive composition containing a styrene type block copolymer in addition to the polyisobutylene and hydrocolloids as taught by Doyle et al. in U.S. Patent No. 4,551,490. The particular adhesive selected used may depend upon medical necessity, such as allergies or upon patient preference.

On the non-adhesive side of film 62 a plastic attachment ring 66 is welded. This ring is used to affix the pouch to the wafer. This may be accomplished in a nondetachable manner, so as to form a one piece appliance or in a detachable manner, to create a two piece applicance, wherein the pouch may be removed for cleaning or replacement without removal of the wafer from the body.

The attachment ring 66 on the wafer defines the area within which the stoma receiving opening 68 is situated. As shown in Fig. 9, within the confines of the ring, the opening 68 can be of any size, shape and location.

Likewise, the outline 70 of the wafer can be cut to any configuration, such as the shape illustrated in Fig. 10. Of course, the wafer cannot be smaller than the base 72 of the attachment ring and there must be sufficient surface area for the adhesive to securely adhere.

The stoma receiving opening 68 and outline 70 are cut using a cutting tool mounted on an X-Y table controlled by the control computer. A variety of know technologies can be utilized for this purpose. A high speed grinding/machine tool (preferrably a cone shaped tool is employed to provide a beveled cut which will allow better skin protection near the stoma), a wafer jet cutter, a knife, a saw, a laser cutter, a hot wire or an RF scapel can be used. A nibbling or punching technique may also work well.

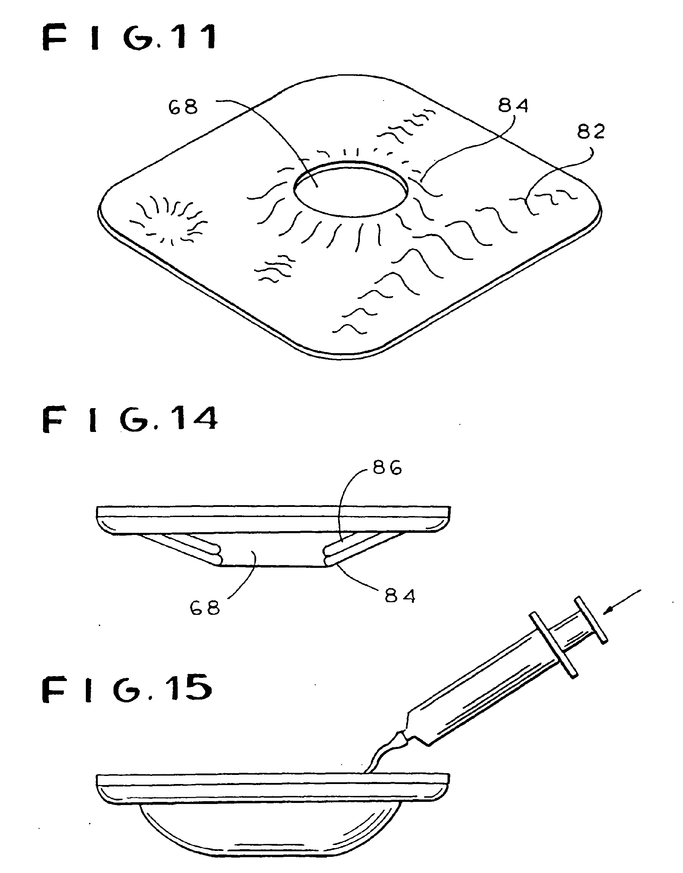

As illustrated in Figure 11, the surface contour of the wafer may be adjusted to conform with skin surface irregularities such as folds, crevices and depressions. One way to form a wafer with the desired surface contour is by heating and forming the barrier adhesive into a mold. The adhesive may be backed up by other materials which have the necessary filling properties, but are easier to handle and lower in cost than the barrier adhesive.

The filler material may be formed first to the desired shape, and then the barrier adhesive is vacuum formed over it. Methods other than heat may also be employed to form the shaped barrier adhesive, such as UV light curable materials.

The mold that is made to form wafers with a surface contour may be made employing several other techniques as well, including sterolithography, machining by computer aided manufacturing (CAD/CAM) process or by a sintered powdered metal technique.

Another method is to use a flexible array of pins. The array of pins forms the shaped mold by having pins project into the mold to a degree which is based on an electronic signal. This technique represents a truly flexible approach to tooling.

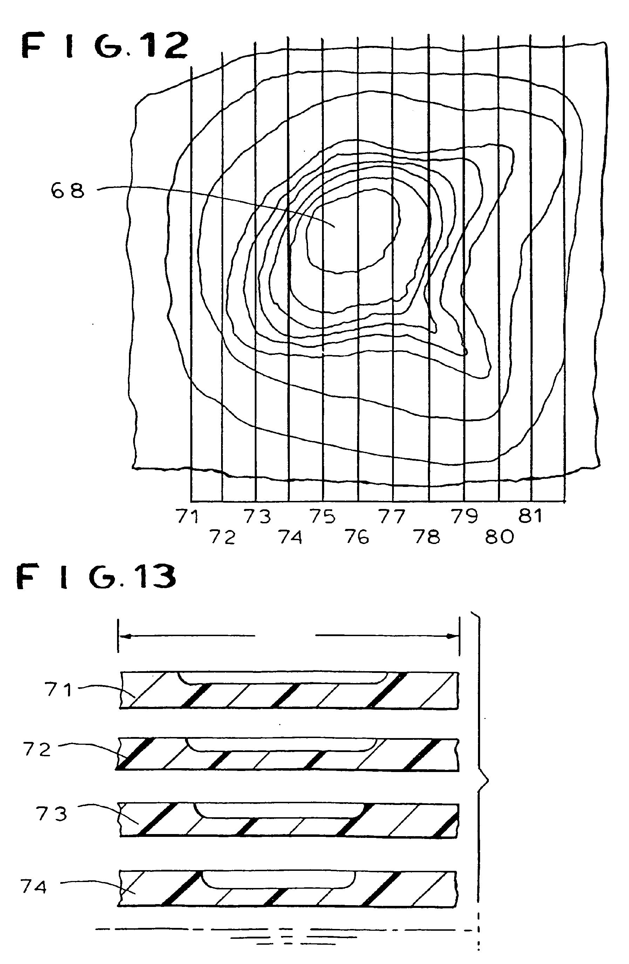

Another method of preparing the mold in accordance with the information in the database is illustrated in Figures 12 and 13. The mold is built by cutting and stacking a number of cross-sectional layers 71-81, one next to the other. Each layer cr slice 71-81 could be formed by cutting a thin sheet into the desired configuration by a water jet cutter or any other suitable instrument. Slices, such as illustrated in Fig 13 are then bonded together to form an approximation of the desired surface contour. The accuracy of the mold increases as the number of slices increases.

Another way to form protrusions 82 on the surface of a wafer is the use of customized parts or sections of particular shapes and sizes which are positioned and affixed to the surface of the film prior to coating with the adhesive. Such sections can stick on or snap into place on the film.

It has been previously recognized that providing the wafer with a convex surface 84 results in a substantially improved seal with the skin for stomas of particular topography. Accordingly, it is known to employ an annular rigid on semi-rigid plastic disk 86 with a convex surface as illustrated in Figure 14- between the wafer film and adhesive to provide a wafer with the desired convex surface contour.

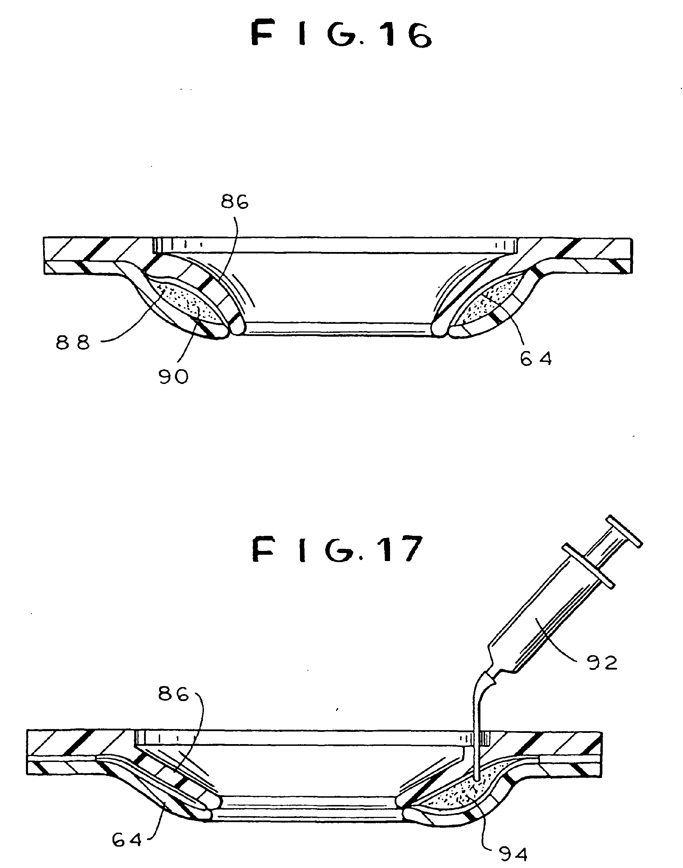

However, it is often desirable to provide the surface contour with a more customized convex shape than the simple conical shape obtained by the use of a conventional convex disk, as shown in Fig. 15. Accordingly, additional material, in the form of a powder or liquid, can be used to build up the convex surface to have the desired contour. This technique is illustrated in Figs. 16 to 23.

An annular shaped hollow pouch 88 is affixed by adhesive or the like to the convex surface of the disk 36. In Fig. 16, the pouch 88 chamber is filled with corn starch 90 . The wafer is then coated with adhesive 64. The adhesive 64 can be manipulated to obtain the desired surface contour. The corn starch 90 will hold the shape.

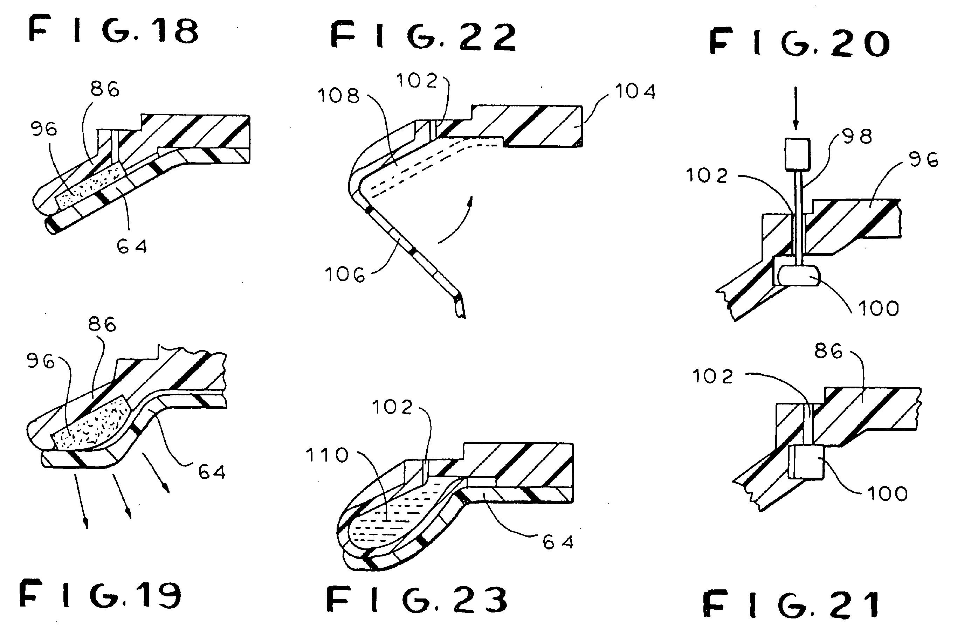

Alternately, as illustrated in Fig. 17, a syringe 92 can be used to inject water, oil or a quick setting resin 94 into the annular pouch cavity. In Fig. 18 a compressed sponge or "fizzies" 96 is situated within the cavity. Water is then injected into the cavity to expand the material to the desired extent, as illustrated in Fig. 19.

As seen in Figs. 20 and 21, the fluid can be injected through a hollow metal needle 98 of the type used to inflate footballs and basketballs. Embeded within the convex disk 86 is a compressible valve clyinder 100 which is compressed by the needle (Fig. 20) to allow fluid into the chamber, but seals the inlet opening 102 when the needle is removed (Fig. 21).

Figures 22 and 23 illustrate a method of forming an intergral chamber as part of the convex disk. As shown in Fig. 22, disk 104 is formed with a cover portion 106 which is bent and sealed into position (shown in phantom in Fig. 22) so as to form chamber 108. Thereafter, liquid 110 is injected through opening 102 to form the required curvature and adhesive 64 is deposited on the curved surface.

### CUSTOMIZATION OF THE COLLECTION POUCH

The pouch has a front barrier film wall and a rear barrier film wall. The walls are made of thin, flexible film which is heat welded around the periphery to form an enclosed receptacle. Depending upon the type of stomal discard, the pouch may include drainable outlet which is sealed with a clip or it may include a liquid drainable tap valve. Otherwise, the bottom may be sealed in the same manner as periphery.

The films from which the pouch walls may be made are selected from materials which possess the properties of being moisture impermeable, odor impermeable and capable of being heat sealed or impulse welded. Suitable materials include polyethylene, copolymers of polyethylene and ethylene vinyl acetate, co-polymers of vinyl chloride and polyvinylidene chloride and laminates thereof. The pouch walls are preferably from about two to four mils thick.

Pouches may be customized by moving the tail location, changing the outline, changing the attachment ring location, changing the flange type, changing the filter and tap location or the tap type. These changes can be achieved in a number of different ways.

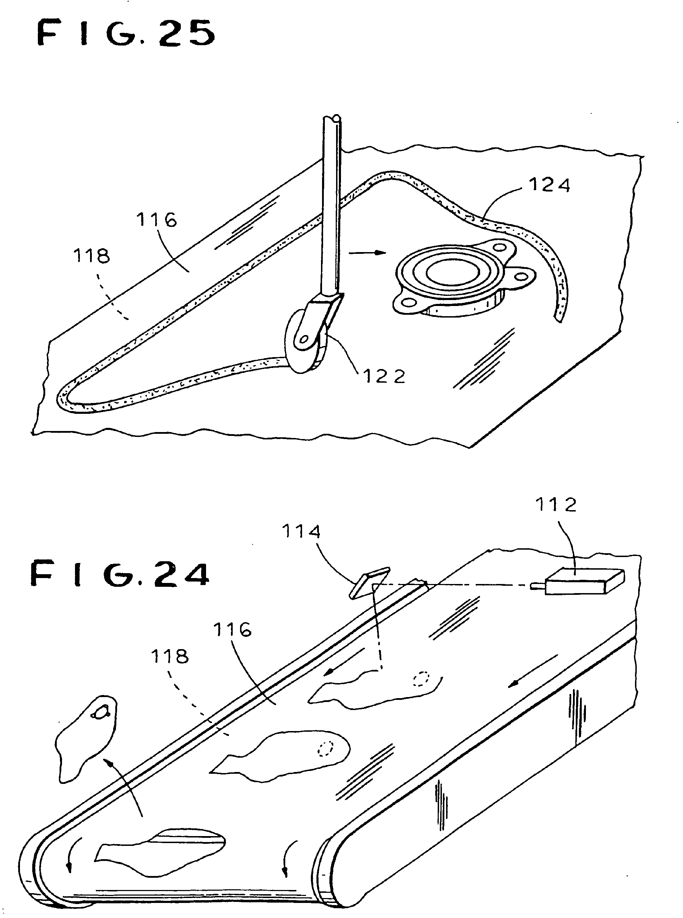

The outline may be welded by using a laser beam, as illustrated in Fig. 24 or a hot roller mounted on a X-Y table, as illustrated in Fig. 25. Alternately, the weld can be formed ultrasonically or by a hot air weld nozzle.

As shown in Fig. 24, the laser 112 generates a beam which is reflected by a mirror 114 onto two sheets of barrier film 116, 118, situated one on top of another, on a conveyer table 120. The mirror 114 is moved in accordance with commands from the controller to cut and seal pouches with the desired outline. This process has the advantage of having no fixed tooling and no limit on the pouch size and shape.

Fig. 25 illustrates the hot wheel sealing approach. The movement of the wheel 122 is guided by the controller to seal the films along outline 124. This method is extremely flexible and provides good control over temperature, time and pressure. It is similar to that which is presently used in vinyl fabrication.

Flexible outline weld tools that bend to the needed shapes as directed by an electronic signal may be employed. Another possibility is the use of rigid sections of outline weld tools that are selected to be combined with others to form customized outlines. If a limited number of custom outlines are required, a specific outline tool may be selected by the machine from a rack or rotary table as needed.

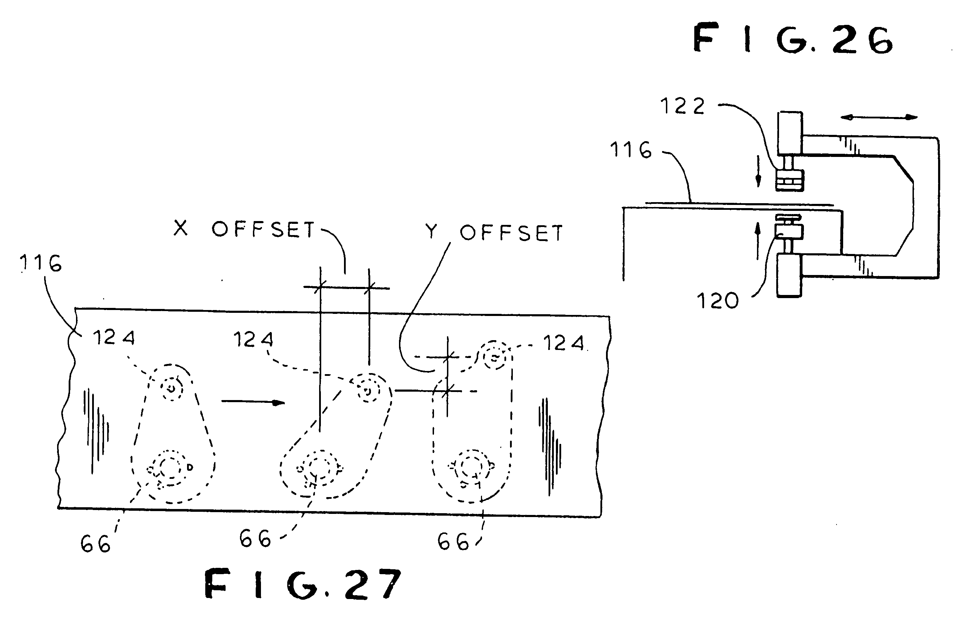

Prior to welding the pouch periphery, the attachment ring, tap and filter welds are made. Fig. 26 shows the welder itself, which includes a support 120 for the part, situated beneath the film 116 and a weld unit 122 supported above the film. The unit is moveable along an X-Y axis so as to position the parts as required relative to the film. Fig. 27 shows the film from above and illustrates that the attachment ring 66 and tap washer 124 may be aligned along the vertical axis of the pouch, offset along the X direction or offset along the Y direction, as illustrated.

In this regard, placement of the components could be achieved by robotic indexing machines. The center of the stoma receiving hole could be used as a reference. Variable index distance may be desireable.

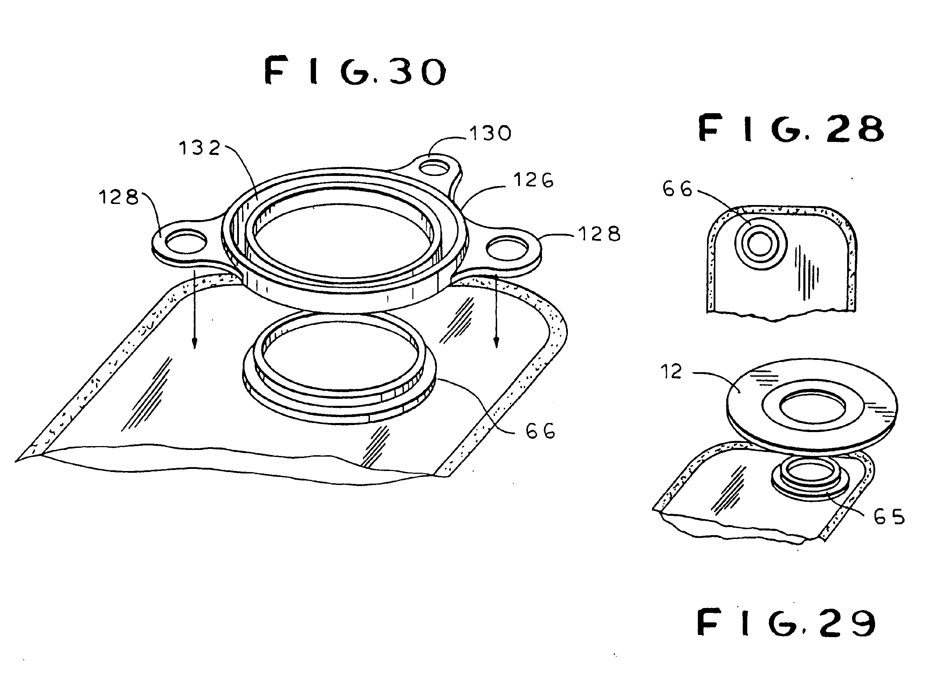

One of the features which is of concern in designing pouches is the "head room", which is the space between the attachment ring and the top edge of the pouch. It is usually desireable to minimize the head room, as shown in Fig. 28 which shows a pouch with the attachment toward the left side. This can be accomplished in the present invention by employing a single type of attachment ring which will act as a permanently welded base and which is designed to accept a variety of different components, depending upon whether the pouch is to be attached to a belt, is to be detachably coupled to the wafer or is to be non-detachably coupled to the wafer.

Figure 29 illustrates a pouch with an attachment ring 66 near the right side which is designed to directly receive a wafer 12 permanently affixed thereto. In this way, ring 66 can be used to form a one piece applicance.

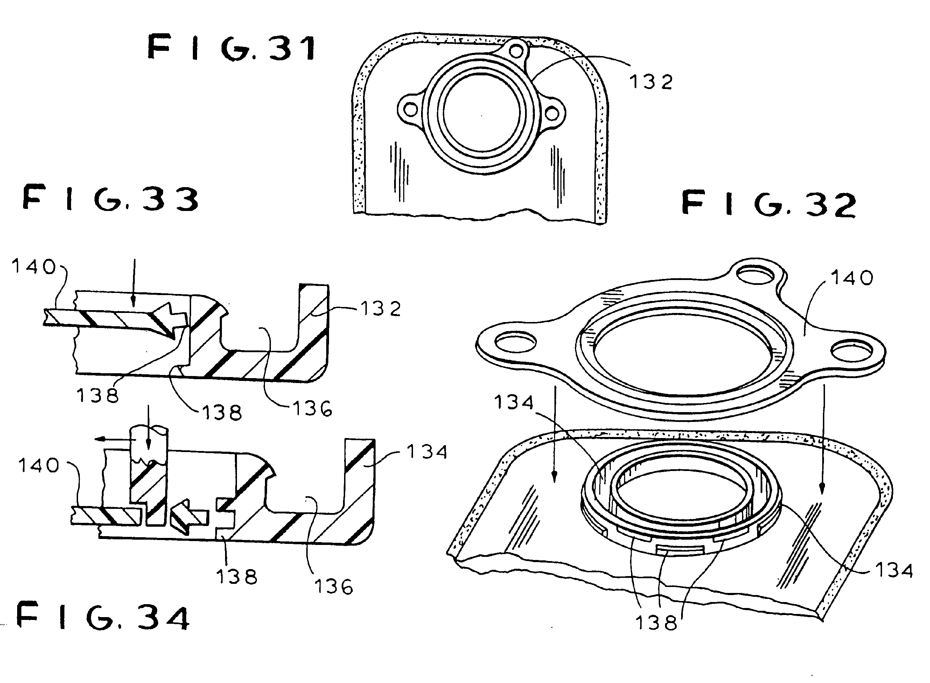

Figure 30 shows the same pouch and ring 66 but in this case a flange 126 is attached thereto. Flange 126 has belt receiving lugs 128 and a burp tab 130. Flanges of different configurations can be used for different applications. As illustrated, flange 126 has an annular channel 132 for detachable coupling with a mating pouch ring. Flange 132 can be welded to ring 66, as shown in Fig. 31. It can have an intergral channel 136 and snap-fit into place on a ring 134, as shown in Fig. 32.

Figs. 33 and 34 illustrate two different attachment ring cross-sections. Each ring 134 has a "U" shaped channel 136 with a series of spaced protrusions 138 on the exterior wall.

A flange 140 with lugs for belt attachment and/or a burp tab can be positioned over the pouch attachment ring in a snap-in fashion, where the projections on the exterior wall of the ring have a tapered structure, as shown in Fig. 33. Alternatively, a stretch and release method could be used where the resiliency of the plastic flange material is utilized, as indicated by the arrows in Fig. 34.

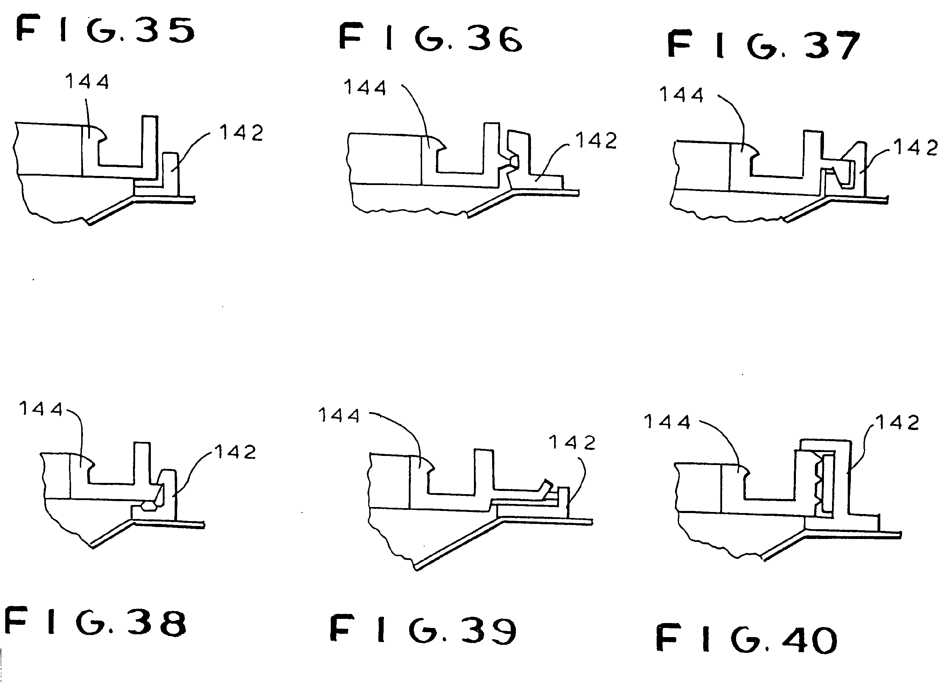

Another alternative is to employ a two part attachment ring, as illustrated in Figs. 35 through 40. Here a first part 142 of the ring is welded directly and permanently to the pouch wall and a second part 144 of the ring, which includes the annular channel, is joined to the first part by adhesive, as seen in Fig. 35, by a radially outwardly extending protrusion with a sealing ring, as shown in Fig. 36, by a "L1" shaped protrusion, as shown in Fig. 37, a snap undercut structure, as shown in Fig. 38, a hook and loop ribbon fastener type attachment, as seen in Fig. 39 or a ribbed wall with a flat seal, as shown in Fig. 40.

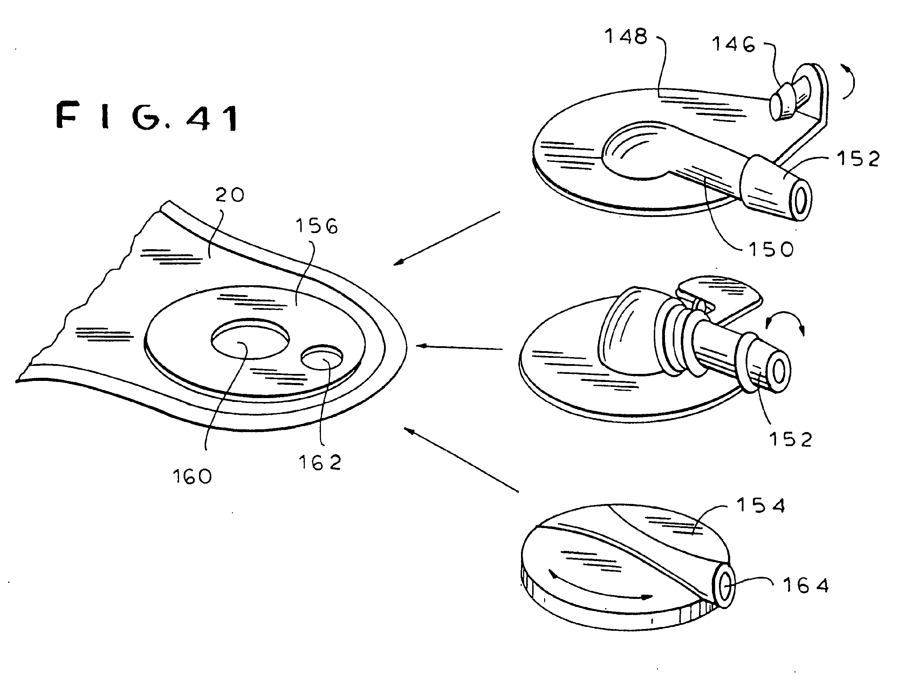

As mentioned above, the tap style can also be customized. Fig. 41 illustrates three different tap styles. At the top is shown the fold over type, where the plug 146 is affixed to the base 148. The flexible outlet 150 is provided with a tapered sleeve 152 to accept the plug 146 in a sealing manner. The middle illustrates the Accuseal® type, where the tube 152 is rotated to seal. The bottom illustrates the rotary type, where the base 154 is rotated between open and closed positions.

All three tap types can be mounted to the pouch using a unique weld tap washer 156. Washer 156 has first and second knock out slugs which are selectively removed to suit the desired tap style.

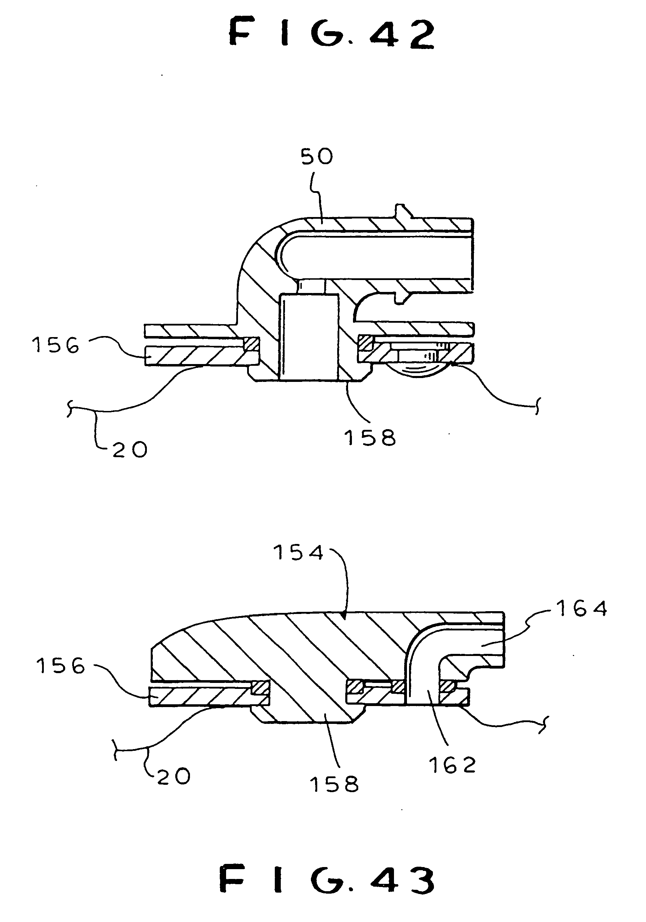

As seen in Figs. 42 and 43, each tap style has a central downward protruding post 158 which snap fits into the center opening 160 in washer 156. If the tap is of the foldover or Accuseal® type, post 158 is hollow and defines a conduit connecting the pouch interior with the tap tube, as seen in Fig. 42. The washer body covers the second opening 162 in the washer which is closed at the bottom by the pouch wall.

If a rotary type tap is employed, as seen in Fig. 43, the central post 158 is solid and acts to define the axis about which the tap rotates. The tube 164 terminates above the position of the second opening 162 and when in the appropriate rotational position, will align with the second opening so as to provide a channel to the pouch interior, as seen in Figure 43.

Certain pouches have no tap but instead an open bottom which is closed when in use by a tail clip. This feature can be customized by tailoring the length of the clip to match the width of the pouch outlet.

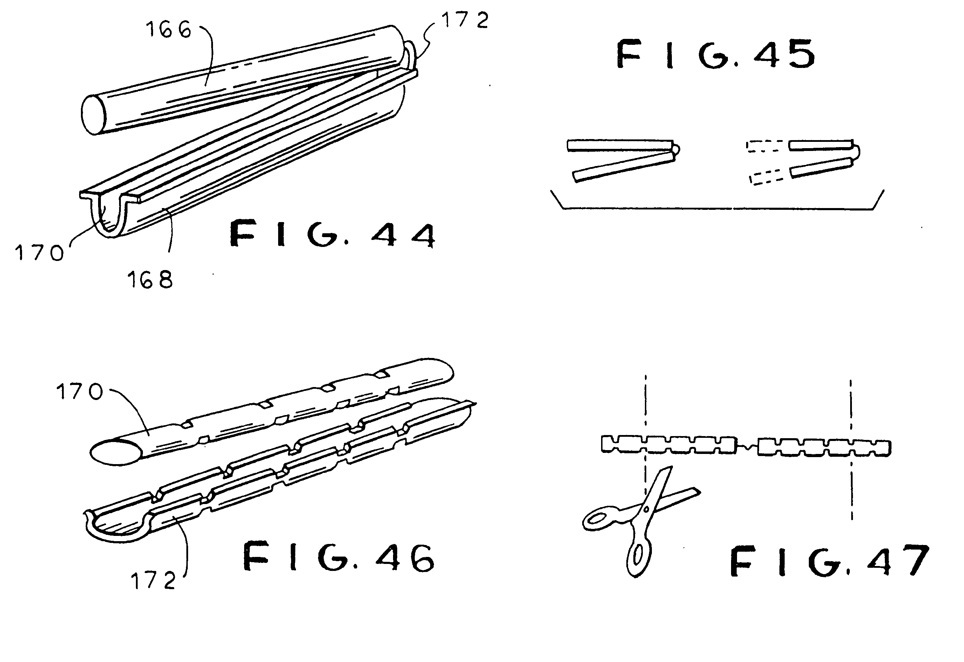

Fig. 44 shows a tail clip which consists of a bar 166 and a "U" shaped member 168 with a bar receiving channel 170. The parts are attached to each other by a resilient part 172 which maintains alignment between the parts. The pouch tail is wrapped around bar 166 and the bar is then snapped into the member 168 to close the tail. The parts can be cut to the appropriate size, as seen in Fig. 45.

Fig. 46 shows a second embodiment where the bar 170 and receiving member 172 are notched to facilitate cutting both parts to equal lengths to match the tail width, as seen in Fig. 47.

It will now be appreciated that the present invention relates to customized ostomy devices including wafers and pouches of the two piece detachable type. Detailed measurements of the topography of the stoma area are taken and recorded. These measurements are used to form an electronic 3-dimensional image or map of the anatomy of the patient. This image, user history and preferences are maintained in a patient file databse.

When devices are to be produced, the information in the patient's file is downloaded into a computer which analyzes the information and selects the physical attributes of the wafer, including the adhesive type, stoma receiving opening size, shape and location, the outline and structure of the pouch, including the wall film, colar, tail location, and type of attachment ring, tab style, tab position and filter.

The computer then causes the fabrication equipment to select the appropriate materials and tools, to make the necessary adjustments and guide the tools to produce wafers and pouches to suit the specific requirements of the user. Accessory selection, packaging and shipping is also in accordance with the user's needs.

While only a limited number of emodiments of the present invention are disclosed for purposes of illustration, it is obvious that many variations and modifications could be made thereto. It is intended to cover all of these variations and modifications which fall within the scope of this invention, as set forth in the following claims:

## Claims

1. A two-piece ostomy device customized for a particular user comprising an adhesive wafer (18) with a stoma receiving opening, an attachment ring defining the area in which said opening is situated, a waste collection pouch (20) with an inlet, means (26) for mounting said pouch (20) on said wafer (18) with said inlet aligned with said opening
**characterised in that**
the device is a two-piece device obtainable by fabricating the wafer (18) separated from the pouch (20) so that the size, shape and location of said opening within said area is selected in accordance with the size, shape and location of the stoma of the user.

2. The device of claim 1 **characterised in that** the size, shape and location of said opening is determined by utilizing electronic information obtained by measuring the surface or topography of the stomal area of the patient, recording information relating to the measurements of the stomal area and transforming the recorded information into electronic form.

3. The device of any preceding claim **characterised in that** the shape of the opening is irregular.

4. The device of any preceding claim **characterised in that** said wafer (18) has a shape and size selected in accordance with the topography of the stomal area of the user.

5. The device of claim 4 **characterised in that** the shape of the wafer (18) is irregular.

6. The device of any preceding claim **characterised in that** the pouch (20) has a contour selected in accordance with the topography of the stomal area of the user.

7. The device of claim 6 **characterised in that** the contour of the pouch (20) is irregular.

8. The device of any preceding claim **characterised in that** said pouch (20) comprises an attachment ring (26) and **in that** the location of said attachment ring on said pouch (20) is selected in accordance with the topography of the stomal area of the user.

9. The device of any preceding claim **characterised in that** the surface contour of said wafer (18) is selected in accordance with the topography of the stomal area of the user.

## Patentansprüche

1. Für einen speziellen Benutzer angepasste zweiteilige Ostomievorrichtung, die aufweist: eine Klebstoffscheibe (18) mit einer stomaaufnehmenden Öffnung, einen Befestigungsring, der den Bereich definiert, in dem sich die Öffnung befindet, einen Abfallprodukt-Sammelbeutel (20) mit einem Einlass, einer Einrichtung (26) zum Montieren des Beutels (20) an die Scheibe (18), wobei der Einlass zur Öffnung ausgerichtet ist,
**dadurch gekennzeichnet, dass**
die Vorrichtung eine zweiteilige Vorrichtung ist, die durch Fertigen der Scheibe (18) getrennt vom Beutel (20) so erlangbar ist, dass die Größe, Form und Lage der Öffnung im Bereich in Übereinstimmung mit der Größe, Form und Lage des Stomas des Benutzers ausgewählt ist.

2. Vorrichtung nach Anspruch 1, **dadurch gekennzeichnet, dass** die Größe, Form und Lage der Öffnung durch Nutzung elektronischer Informationen bestimmt ist, die durch Messen der Oberfläche oder Topografie des Stomabereichs des Patienten, Aufzeichnen von Informationen, die die Messungen des Stomabereichs betreffen, und Transformieren der aufgezeichneten Informationen in elektronische Form erhalten werden.

3. Vorrichtung nach einem der vorstehenden Ansprüche, **dadurch gekennzeichnet, dass** die Form der Öffnung unregelmäßig ist.

4. Vorrichtung nach einem der vorstehenden Ansprüche, **dadurch gekennzeichnet, dass** die Scheibe (18) eine Form und Größe hat, die in Übereinstimmung mit der Topografie des Stomabereichs des Benutzers ausgewählt sind.

5. Vorrichtung nach Anspruch 4, **dadurch gekennzeichnet, dass** die Form der Scheibe (18) unregelmäßig ist.

6. Vorrichtung nach einem der vorstehenden Ansprüche, **dadurch gekennzeichnet, dass** der Beutel (20) eine Kontur hat, die in Übereinstimmung mit der Topografie des Stomabereichs des Benutzers ausgewählt ist.

7. Vorrichtung nach Anspruch 6, **dadurch gekennzeichnet, dass** die Kontur des Beutels (20) unregelmäßig ist.

8. Vorrichtung nach einem der vorstehenden Ansprüche, **dadurch gekennzeichnet, dass** der Beutel (20) einen Befestigungsring (26) aufweist, und dadurch, dass die Lage des Befestigungsrings auf dem Beutel (20) in Übereinstimmung mit der Topografie des Stomabereichs des Benutzers ausgewählt ist.

9. Vorrichtung nach einem der vorstehenden Ansprüche, **dadurch gekennzeichnet, dass** die Oberflächenkontur der Scheibe (18) in Übereinstimmung mit der Topografie des Stomabereichs des Benutzers ausgewählt ist.

## Revendications

1. Dispositif d'ostomie en deux pièces adapté à un utilisateur particulier comprenant une plaquette adhésive (18) avec une ouverture de réception de stomie, une bague de fixation définissant la zone dans laquelle ladite ouverture est située, une poche de collecte de déchets (20) avec une entrée, un moyen (26) pour installer ladite poche (20) sur ladite plaquette (18), ladite entrée étant alignée avec ladite ouverture,
**caractérisé en ce que**
le dispositif est un dispositif en deux pièces pouvant être obtenu en fabricant la plaquette (18) séparément de la poche (20) de sorte que la taille, forme et emplacement de ladite ouverture dans ladite zone est sélectionnée en accord avec la taille, la forme et l'emplacement de la stomie de l'utilisateur.

2. Dispositif selon la revendication 1, **caractérisé en ce que** la taille, la forme et l'emplacement de ladite ouverture est déterminée en utilisant des informations électroniques obtenues en mesurant la surface ou la topographie de la zone de stomie du patient, en enregistrant les informations se rapportant aux mesures de la zone de stomie et en transformant les informations enregistrées en forme électronique.

3. Dispositif selon l'une quelconque des revendications précédentes, **caractérisé en ce que** la forme de l'ouverture est irrégulière.

4. Dispositif selon l'une quelconque des revendications précédentes, **caractérisé en ce que** ladite plaquette (18) a une forme et une taille sélectionnées en accord avec la topographie de la zone de stomie de l'utilisateur.

5. Dispositif selon la revendication 4, **caractérisé en ce que** la forme de la plaquette (18) est irrégulière.

6. Dispositif selon l'une quelconque des revendications précédentes, **caractérisé en ce que** la poche (20) a un contour sélectionné avec accord avec la topographie de la zone de stomie de l'utilisateur.

7. Dispositif selon la revendication 6, **caractérisé en ce que** le contour de la poche (20) est irrégulier.

8. Dispositif selon l'une quelconque des revendications précédentes, **caractérisé en ce que** ladite poche (20) comprend une bague de fixation (26), et **en ce que** l'emplacement de ladite bague de fixation sur ladite poche (20) est sélectionné en accord avec la topographie de la zone de stomie de l'utilisateur.

9. Dispositif selon l'une quelconque des revendications précédentes, **caractérisé en ce que** le contour de surface de ladite plaquette (18) est sélectionné en accord avec la topographie de la zone de stomie de l'utilisateur.
